# EUROPEAN PATENT SPECIFICATION

(11) **EP 3 652 147 B1**
(45) Date of publication and mention of the grant of the patent: **04.11.2020**
(21) Application number: 18779660.2
(22) Date of filing: 28.09.2018
(51) Int. Cl.: C07C 213/06

(54) **PROCESS FOR PREPARING DIMETHYLAMINOALKYL (METH)ACRYLATES**
VERFAHREN ZUR HERSTELLUNG VON DIMETHYLAMINOALKYL(METH)ACRYLATEN
PROCÉDÉ DE PRÉPARATION DE DIMÉTHYLAMINOALKYL (MÉTH)ACRYLATES

(30) Priority: 04.10.2017 DE 102017217620
(43) Date of publication of application: 20.05.2020
(73) Proprietor: Evonik Operations GmbH, 45128 Essen (DE)
(72) Inventor: TRESKOW, Marcel, 64293 Darmstadt (DE); KRÜGER, Torsten, 64405 Messbach (DE); SCHÜTZ, Thorben, 64665 Alsbach-Hähnlein (DE); KRILL, Steffen, 64367 Mühltal (DE)
(74) Representative: Evonik Patent Association
(86) International application number: PCT/EP2018/076447
(87) International publication number: WO 2019/068578

(56) References cited:
- GB-A- 2 162 516
- US-A- 4 672 105
- US-B2- 6 706 910

## Description

The present invention relates to a process for preparing dimethylaminoalkyl (meth)acrylates from alkyl (meth)acrylate and dimethylaminoalkanol. Described herein is the use of a catalyst system comprising a solution of a lithium alkoxide in alcohol in the preparation of a dimethylaminoalkyl (meth)acrylate.

The preparation of 2-dimethylaminoethyl methacrylate using the catalyst LiNH₂ is known from the prior art. The catalyst LiNH₂ is a powder which is virtually insoluble in all organic solvents, tends to cake, and induces polymerization. For a simplified regime with reduced downtime, greater facility for metering, and fewer disruptions, therefore, there was a need for liquid catalysts which exhibit high solubility and, ultimately, activity in the reaction matrix.

DE 1 965 308 describes carrying out the transesterification between dimethylaminoethanol and alkyl acrylates or methacrylates, such as methyl methacrylate, at defined ratios of the reactants, using sodium methoxide or potassium methoxide as catalyst, and using inhibitors to delay the polymerization of the methyl methacrylate and of the end product. The catalyst is added gradually over the course of time, in order thereby to improve the yields, particularly space-time yields. Because of the continuous supplying of the catalyst during the reaction time, and the tendency of sodium methoxide and/or potassium methoxide to cause secondary reactions and unwanted polymeric products, this technology is likewise not entirely satisfactory on a commercial basis.

DE 3 423 441 A1 discloses a process for preparing esters of acrylic or methacrylic acid with alcohols by transesterification of the acrylic or methacrylic esters of C1 to C4 alcohols with different alcohols, with the exception of polyhydric alcohols, the transesterification reaction being carried out in the presence of a catalyst system consisting of a calcium halide or calcium oxide and an organolithium compound. A disadvantage of the process described is that the presence of halides generally leads to the formation of organically bonded halogen, through addition compounds. Where halides are present, the reaction residue cannot generally be burned, because of the risk of formation of dioxins during burning - in the presence of chlorides, for example, the especially toxic 2,3,7,8-tetrachlorodibenzodioxin (CAS 1746-01-6).

There therefore continues to be a need for processes for preparing dimethylaminoalkyl (meth)acrylates that enable rapid reaction times with minimal secondary reactions and by-products.

It has now surprisingly been found that the aforesaid disadvantages of the prior art can be overcome if a catalyst system is used that comprises a solution of a lithium alkoxide in alcohol.

In a first aspect, therefore, the present invention relates to a process for preparing dimethylaminoalkyl (meth)acrylate which is characterized in that a mixture comprising (a) alkyl (meth)acrylate, (b) dimethylaminoalkanol and (c) a catalyst system comprising a solution of a lithium alkoxide in alcohol is reacted, wherein the catalyst system contains no alkaline earth metal compounds.

With the process of the invention it is possible to realize comparatively high temperatures and hence quicker reaction times.

The reaction scheme below represents by way of example the preparation of dimethylaminoethyl methacrylate from methyl methacrylate and 2-dimethylamino-1-ethanol:

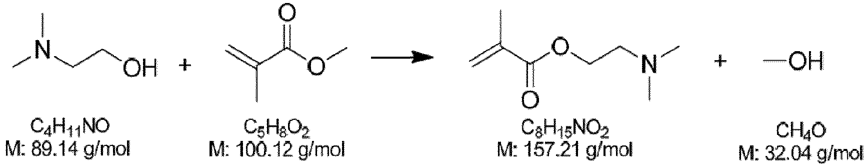

The process of the invention, however, is not confined to the use of 2-dimethylamino-1-ethanol. Examples of other dimethylaminoalkanols which can be used in accordance with the invention are 3-dimethylamino-1-propanol, 4-dimethylamino-1-butanol, 5-dimethylamino-1-pentanol, 6-dimethylamino-1-hexanol, 7-dimethylamino-1-heptanol, and 8-dimethylamino-1-octanol. Preferred in accordance with the invention is the use of 2-dimethylamino-1-ethanol.

Similarly, the process of the invention is not confined to the use of methyl methacrylate. Examples of further methacrylates which can be used in accordance with the invention are ethyl methacrylate, propyl methacrylate, butyl methacrylate, pentyl methacrylate, hexyl methacrylate, heptyl methacrylate, and octyl methacrylate. Acrylates which can be used in accordance with the invention are methyl acrylate, ethyl acrylate, propyl acrylate, butyl acrylate, pentyl acrylate, hexyl acrylate, heptyl acrylate, and octyl acrylate. Preferred in accordance with the invention is the use of methyl methacrylate.

The scheme shown above and also the breadth of the aminoalkyl alcohols which can be used encompasses likewise, as is apparent to the skilled person, the preparation of the corresponding acrylate compounds, in which case methyl methacrylate is a preferred starting compound.

The catalyst system used in accordance with the invention comprises a solution of a lithium alkoxide in alcohol and contains no alkaline earth metal compounds. Examples of lithium alkoxides for use in accordance with the invention are lithium methoxide, lithium ethoxide, lithium n-propoxide, lithium *iso*-propoxide, lithium n-butoxide, lithium *iso*-butoxide and lithium *tert*-butoxide. Examples of alcohols for use in accordance with the invention are methanol, ethanol, n-propanol, *iso*-propanol, n-butanol, *iso*-butanol and *tert*-butanol. Lithium alkoxide and alcohol can in principle be selected independently of one another.

It is particularly preferred in accordance with the invention for the catalyst system to consist of a solution of lithium methoxide in methanol or a solution of lithium *tert*-butoxide in methanol or *tert-*butanol.

It is also in accordance with the invention for the catalyst system to contain no alkaline earth metal compounds, more particularly no calcium compounds.

In general the solution of lithium alkoxide in alcohol contains 5 to 15 wt% of lithium alkoxide, for example 6 to 14 wt%, 7 to 13 wt%, 8 to 12 wt%, 9 to 11 wt%, or 10 wt% of lithium alkoxide, based on the alcohol. The weight percentage ranges reported here are valid for any combination of the above-specified lithium alkoxides with any of the above-specified alcohols.

The fraction of lithium alkoxide in the reaction mixture is advantageously 0.4 to 5.0 mol%, for example 1.0 to 4.5 mol%, 1.5 to 4.0 mol%, 2.0 to 3.5 mol%, 2.5 to 3.0 mol%, based on the dimethylaminoalkanol. The molar percentage ranges reported here are valid for any combination of the above-specified lithium alkoxides with any of the above-specified dimethylaminoalkanols.

The stated stoichiometric catalytic amounts of the active catalyst are based on the amount of the aminoalkyl alcohol ultimately consumed. The lithium alkoxide solution may be added at the start of the reaction, so that the total amount is present when the reaction is commenced. It is also possible to include at least part of the amount of catalyst in the initial charge and then to make successive additions during the reaction.

The use of an excess of the alkyl acrylate or alkyl methacrylate above the amounts required stoichiometrically for the transesterification is advantageous. Generally speaking, a 1.1- to 3.5-fold excess over the stoichiometrically calculated amount is employed. If the batches are increased beyond the laboratory scale, it will tend to be advisable to reduce the excess of alkyl (meth)acrylate. Accordingly, the molar ratio of (a) alkyl (meth)acrylate to (b) dimethylaminoalkanol in the reaction mixture is in the range from 3.5:1 to 1.1:1, for example in the range from 3.4:1 to 1.2:1, 3.3:1 to 1.3:1, 3.2:1 to 1.4:1, 3.1:1 to 1.5:1, 3.0:1 to 1.6:1, 2.9:1 to 1.7:1, 2.8:1 to 1.8:1, 2.7:1 to 1.9:1, 2.6:1 to 2.0:1, 2.5:1 to 2.1:1, 2.4:1 to 2.2:1, or 2.3:1.

In general there is no need to use a solvent as well. Optionally, however, it is also possible for inert (non-radical-forming) solvents to be used. Examples would include hydrocarbons, such as cyclohexane, n-hexane or heptane, and toluene.

In order to suppress polymerization by-products of the alkyl (meth)acrylate and also of the aminoalkyl (meth)acrylates that form, it is advisable to include a stabilizer (radical scavenger). Serving as stabilizers may be all of the radial scavengers known from the prior art, examples being hydroquinone compounds, thio compounds, or amines. A comprehensive description of suitable stabilizers is found, for example, in H. Rauch-Puntigam, Th. Völker: "Acryl- und Methacrylverbindungen", Springer-Verlag, 1967. In order to obtain ideal outcomes in terms of suppression of polymerization and boosting of the dimethylaminoalkyl (meth)acrylate yield and purity, the inhibitor preferred is phenothiazine alone or in combination with N,N-diethylhydroxylamine. The combination is particularly useful because phenothiazine is a solid and therefore represents the effective inhibitor in the tank, whereas diethylhydroxylamine is a liquid and therefore represents the effective inhibitor in the column. Other inhibitors are methyl ethers of hydroquinone, nitrobenzene, di-*tert*-butylcatechol, hydroquinone, *p*-anilinophenol and di(2-ethylhexyl) octylphenyl phosphite. For applications where the dimethylaminoalkyl (meth)acrylate to be produced will be subjected to subsequent homopolymerization or copolymerization with other polymerizable monomers, the inhibitor should not reduce the activity of the polymerization. A preferred storage inhibitor for dimethylaminoalkyl (meth)acrylate to be used in this way is the methyl ether of hydroquinone.

Preferably, in accordance with the invention, the reaction mixture comprises one or more inhibitors selected from the group consisting of hydroquinone monomethyl ether and 2,4-dimethyl-6-tertbutylphenol.

The reaction is carried out usefully at above room temperature, preferably in the range from 100 to 140°C, for example in the range from 105 to 135°C, 110 to 130°C, 115 to 125°C, or at 120°C. In one preferred embodiment of the process of the invention, the reaction mixture is heated to a temperature of at least 100°C.

Where the reaction is carried out in a cascade of two or more stirred tanks ("stirred tank cascade"), the temperature regime may be such, for example, that the same temperature is set in each tank in the cascade. Alternatively to this, the temperature regime may also be such that the temperature increases along the cascade, i.e. that a temperature gradient is set. In one preferred embodiment of the process of the invention, the reaction is carried out in a cascade consisting of 2 to 5 stirred tanks, more preferably in a cascade consisting of 3 stirred tanks, with a temperature of 100°C being set in the first tank in the cascade and a temperature of 140°C being set in the last tank in the cascade.

The total reaction times are generally 5 to 20 hours, in many cases 6 to 12 hours. As a guide to the duration of the alcoholysis itself, it is possible in the majority of cases to assume 3 ± 1 hours, plus around 2 to 3 hours for the remainder of the time until the end of the reaction; here, however, the size of the batches is also a factor. The methanol formed in the transesterification from methyl (meth)acrylate may usefully be drawn off in the azeotropic mixture with the dimethylaminoalkyl (meth)acrylate, at 65 to 75°C, for example.

The reaction may be carried out, for example, as follows: 2-dimethylamino-1-ethanol is charged to a suitable reaction vessel together with the excess methyl methacrylate and the stabilizer. Lithium methoxide as catalyst may be added during the reaction, or is present from the start. The reaction mixture is brought with stirring to reaction temperature; for example, it is heated to boiling when using methyl methacrylate. The methanol which forms is advantageously drawn off in the azeotrope with the dimethylaminomethyl (meth)acrylate at overhead distillation temperature of 70°C. At an overhead temperature of up to around 98°C, the remaining methanol is drawn off together with unconverted methyl methacrylate; lastly, the remaining methyl methacrylate is distilled off advantageously under reduced pressure at a maximum sump temperature of 150°C. Working-up takes place in a conventional way. For example, it has proved appropriate to add bleaching earth or activated carbon to the crude ester and to carry out filtration, following brief agitation, using precoat filters or pressure filters.

The yields of the desired transesterification product in the process of the invention are usually of the order of > 80 wt%, often > 90 wt%. Deserving of emphasis are the extremely low fractions of addition compounds to the vinylic double bond, and also other by-products.

The process of the invention can be carried out either batchwise or continuously. "Batchwise" denotes the reaction regime in a reaction tank or reactor. This procedure is often also referred to as a "batch" process. The reactants, in the simplest case methyl methacrylate and the aminoalkyl alcohol, are in this case included at least fractionally in the initial charge at the start of the reaction, and the reaction is then initiated in the presence of at least portions of the catalyst of the invention. In a further embodiment of the process, catalyst solution and also portions of alkyl methacrylate and/or aminoalkyl alcohol can be metered in additionally in the course of the reaction, since certain volumes or quantities of a developing azeotrope of alcohol (methanol) and dimethylaminoalkyl (meth)acrylate leave the reactor. Consequently, in this procedure referred to as "semi-batch", the space-time yield is boosted for a given tank or reactor volume.

"Continuously", in contrast, denotes the reaction regime in a tubular reactor or in a cascade of reaction tanks, where on the one hand reactants are fed continuously and on the other hand product(s) is (are) taken off continuously. In each of the reaction units described, therefore, there is a partial conversion.

Also a subject of the present invention, therefore, is the process described herein, characterized in that during the reaction further amounts of (a) alkyl (meth)acrylate, (b) dimethylaminoalkanol and optionally (c) catalyst system are added to the reaction mixture, and dimethylaminoalkyl (meth)acrylate which forms is removed partly or completely from the reaction mixture.

Described herein is the use of a catalyst system comprising a solution of a lithium alkoxide in alcohol in a transesterification reaction.

According to a preferred use described herein, the catalyst system contains no alkaline earth metal compounds. According to a particularly preferred use described herein, the catalyst system consists of a solution of lithium methoxide in methanol or of a solution of lithium *tert*-butoxide in methanol or *tert*-butanol.

Described herein is the use of a catalyst system comprising a solution of a lithium alkoxide in alcohol in the preparation of a dimethylaminoalkyl (meth)acrylate.
**Figure 1** shows sump temperatures in the preparation of dimethylaminoethyl methacrylate for a batchwise reaction regime using various catalysts.

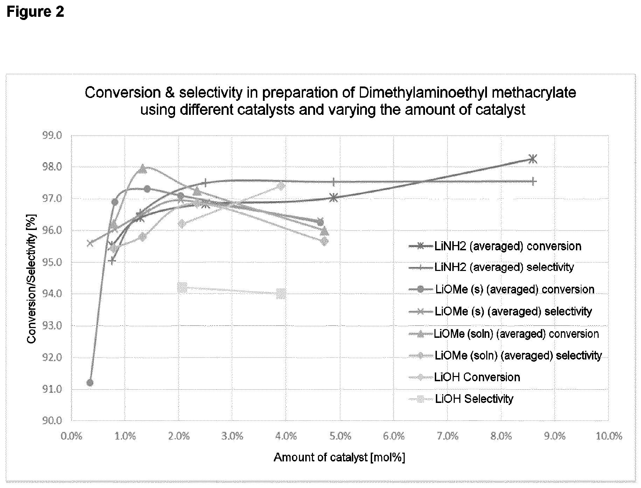

**Figure 2** shows conversion and selectivity in the preparation of dimethylaminoethyl methacrylate for a continuous reaction regime using various catalysts and varying the quantity of catalyst.
The examples which follow serve to elucidate the present invention.

### Examples:

### 1. Batchwise reaction

### Reaction apparatus:

Use was made of a laboratory jack, heating stirrer with external regulation, oil bath (150°C), 0.5-litre and 2-litre four-neck round-bottomed flasks, sump thermometer, air inlet with bubble counter, sabre stirrer, mirrored, vacuum-insulated column with length of 45 cm, packed with 8x8 mm Raschig rings, reflux divider, overhead thermometer, intensive condenser, product condenser, Anschütz-Thiele receiver, distillate receiver.

### Distillation apparatus (work-up):

Use was made of 1-litre three-neck round-bottomed flasks, boiling capillaries, sump thermometer, column with a length of 20 cm, packed with 8x8 mm Raschig rings, distillation bridge, Anschütz-Thiele receiver, distillate receiver, oil bath (130°C), 0 mbar reduced pressure.

### Procedure:

In all the experiments, the reactants, stabilizers and catalysts were included in the initial charge, which was subsequently heated using an oil bath (150°C).

Comparative Examples 1 and 2 could not be completed, owing to an unexpected exothermic reaction.

Inventive Example 3 and Comparative Example 4 were regularly sampled and the theoretical conversion was determined. After the reaction, the excess methyl methacrylate was drawn off and the crude ester remaining in the sump was introduced into a distillation apparatus. The pure 2-dimethylaminoethyl methacrylate was distilled off from the top.

### Comparative Example 1 (potassium methoxide)

### Initial mass:

89.61 g = 1 mol of dimethylaminoethanol 99.48% purity
300.36 g = 3 mol of methyl methacrylate (molar ratio 1:3)
0.1541 g = 980 ppm, based on theoretical yield, of hydroquinone monomethyl ether
0.2358 g = 1500 ppm, based on theoretical yield, of 2,4-dimethyl-6-tert-butylphenol
5.7 g of potassium methoxide, 32% in MeOH = 2.6 mol% based on alcohol

### Comparative Example 2 (sodium methoxide)

### Initial mass:

89.61 g = 1 mol of dimethylaminoethanol 99.48% purity
300.36 g = 3 mol of methyl methacrylate (molar ratio 1:3)
0.1541 g = 980 ppm, based on theoretical yield, of hydroquinone monomethyl ether
0.2358 g = 1500 ppm, based on theoretical yield, of 2,4-dimethyl-6-tert-butylphenol
4.7 g of sodium methoxide, 30% in MeOH = 2.6 mol% based on alcohol

### Inventive Example 3 (lithium methoxide)

### Initial mass:

358.42 g = 4 mol of dimethylaminoethanol, 99.48 % purity
1201.4 g = 12 mol of methyl methacrylate (molar ratio 1:3)
0.6163 g = 980 ppm, based on theoretical yield, of hydroquinone monomethyl ether
0.9433 g = 1500 ppm, based on theoretical yield, of 2,4-dimethyl-6-tert-butylphenol
39.49 g of lithium methoxide, 10% in MeOH = 2.6 mol% based on alcohol

Theoretical yield of 2-(dimethylaminoethyl) methacrylate: 628.8 g

Theoretical yield of 2-dimethylaminoethyl methacrylate: 550.5 g, corresponding to 87.5% of theory

### Comparative Example 4 (lithium amide)

### Initial mass:

358.42 g = 4 mol of dimethylaminoethanol, 99.48 % purity
1201.4 g = 12 mol of methyl methacrylate (molar ratio 1:3)
0.6163 g = 980 ppm, based on theoretical yield, of hydroquinone monomethyl ether
0.9433 g = 1500 ppm, based on theoretical yield, of 2,4-dimethyl-6-tert-butylphenol
2.41 g of lithium amide = 2.6 mol% based on alcohol
Isolated yield of 2-dimethylaminoethyl methacrylate: 628.8 g
Theoretical yield of 2-dimethylaminoethyl methacrylate: 554.6 g, corresponding to 88.2% of theory

### Evaluation:

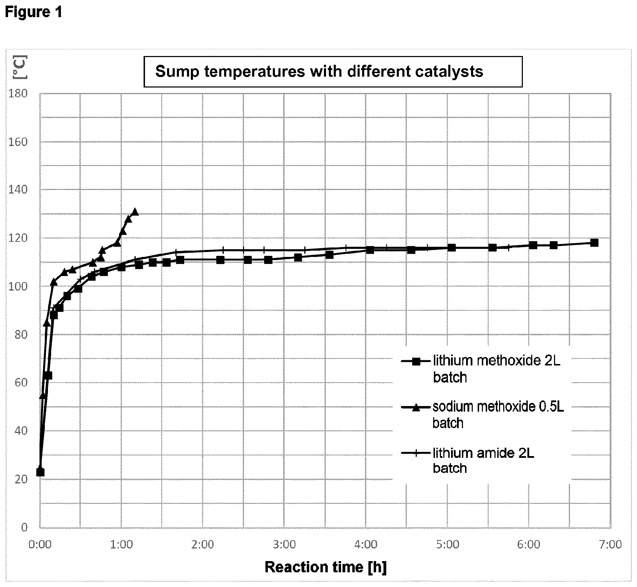

In Comparative Examples 1 and 2, an exothermic reaction (see Figure 1) favoured the formation of oligomers.

In Inventive Example 3 and Comparative Example 4, the transesterification was virtually identical in its course. The main difference here lies in the advantageous use of a liquid catalyst (Inventive Example 3) relative to a solid catalyst (Comparative Example 4).

### 2. Continuous reaction

### Experimental setup:

Use was made of four 100-litre stainless-steel stirring tanks with external half-shells for heating and cooling, connected as a tank cascade, 0.75 m² thin-film evaporator, manufactured by SMS (Buss-SMS-Canzler GmbH, Germany), stainless steel column nominal width 200x6 m with stainless steel Pall rings 20x20 mm, 3 m³ stainless steel circulation evaporator two 3 m³ stainless steel condensers, 1 m² stainless steel final condenser, three 1.7 m³ feed vessels for dimethylaminoalkanol, alkyl (meth)acrylate and N,N-dimethylaminoalkyl (meth)acrylate/alkyl (meth)acrylate mixture from the initial fraction of the purifying distillation, 1 "Lewa H2" metering pump for dimethylaminoalkanol and alkyl (meth)acrylate, 1 "Lewa HL2" metering pump for the initial alkyl (meth)acrylate/dimethylaminoalkanol fraction, powder metering device or pump for solid or liquid catalyst, stabilizing metering via "Lewa HK 2" metering pump, temperature and level control circuit, two 1.2 m³ stainless steel settling vessels, 6 m³ stainless steel crude ester mixing tank, various vapour pressure reducers, temperatures displays.

### Procedure:

The reaction of alkyl (meth)acrylate with dimethylaminoalkanol takes place in three cascade-connected stirred tanks at a sump temperature of 110°C, 120°C and 130°C, respectively. The reaction product from the third stage is passed via a thin-film evaporator. The vapours pass, together with the vapours of stages 1 to 3, via a common line into the bottom third of the column. The dimethylaminoalkanol starting material and the initial fraction are likewise introduced into this column and dewatered by the vapours, which flow in the opposite direction. By rectification, a top product with around 70 % of alcohol, around 29.5% of alkyl (meth)acrylate and around 0.5% of water is brought about. This distillate is free from dimethylaminoalkanol and dimethylaminoalkyl (meth)acrylate. From the column sump dewatered dimethylaminoalkanol and circulation alkyl (meth)acrylate flow into the first reaction stage. A pump conveys the catalyst solution into the first stage. Pure alkyl (meth)acrylate is metered in a further portion via a level regulation system, in accordance with the demand for conversion and alkyl (meth)acrylate content in the crude ester in the column sump. Stabilization is accomplished by dissolving hydroquinone monomethyl ether and/or 2,4-dimethyl-6-tert-butylphenol and/or 4-hydroxy-2,2,6,6-tetramethylpiperidinooxyl (TEMPOL) in alkyl (meth)acrylate and applying this stabilizer solution to the tops of the columns. By this means, the stabilizers are distributed via recycle streams into all process stages and process areas. All stages are sparged with air and operate under atmospheric pressure. The stripped crude ester is cooled to about 40°C, run through settling vessels into a mixing tank, restabilized, and pumped to the storage facility. One settling vessel in each case is in operation, and after around 10 days any solid that has deposited (e.g. lithium methacrylate) is separated off via a centrifuge. The plant permits the throughput of 50 L/h of dimethylaminoalkanol, corresponding to 0.5 kmol/h of dimethylaminoalkyl (meth)acrylate.

For start-up, all of the stages are filled with crude ester and, after heating to reaction temperature, the feeds of dimethylaminoalkanol, alkyl (meth)acrylate, initial fraction and catalyst are applied. Via the circulation evaporator, the column is heated so as to produce around 150 L/h of top product. The distillate is withdrawn only in a quantity such that the composition, with 70% alcohol and 30% alkyl (meth)acrylate remains constant. Monitoring is done by measuring the refractive index (n_{D} 20) or the density, or by using an *in situ* NIR probe. At the 70/30 ratio, it is possible, relative to the 85/15 azeotrope, to remove water at a concentration of around 0.5% overhead and to ensure the required absence of water from the process stages. From the column sump, around 500 L/h of a mixture of 80% alkyl (meth)acrylate, 16% dimethylaminoalkanol and 4% dimethylaminoalkyl (meth)acrylate are run into the 1st stage. The alkyl (meth)acrylate fraction here serves as a circulation product in particular for the dewatering. The prime reason for the different volume flow rates in the column sump and at the top of the column is the difference in calorific data between alkyl (meth)acrylate and alcohol. Because the plant offers no outlet - other than via the crude ester - for dimethylaminoalkanol, GC analysis of the products in the column sump allows, following conversion into mol%, a good overview of conversion and selectivity. This is true both of the crude ester and of the individual reaction stages. The experiments each run over several days, with variations, respectively, in the catalyst and in the catalyst concentration. One of the factors determining the lower limit for the catalyst concentration is the performance range of the metering apparatus. Following emptying and inspection of the reaction chamber, the catalyst is changed.

### Evaluation:

The results are collated in Tables 1 and 2.

LiNH₂, LiOMe (solid) and LiOMe (as a 10 wt% solution in methanol) show results that are similar, and are comparable with LiOH, in terms of the conversion of aminoethanol.

In contrast to LiOH, where crystallizing lithium methacrylate diminishes the transfer of heat owing to formation of deposits, in stages 1 and particularly 2 and 3, and causes a drop in temperature and necessitates weekly cleaning and disconnection, no such deposits are observed in the reaction tanks, within the period of comparison, upon use of LiOMe or LiNH₂. Nevertheless, the ester contains lithium methacrylate in dissolved and precipitated form. The undissolved fraction amounts to approximately 0.1% (relative to 1% in the case of LiOH) and can easily be filtered.

In terms of selectivity for dimethylaminoethyl methacrylate, distinct advantages are apparent relative to LiOH. As a result of formation of high boilers, LiOH exhibits a selectivity of 94% across all the reaction stages. While LiOMe and LiNH₂ do also form the known high boilers in the very first stage (as a result of addition of methanol with dimethylaminoethyl methacrylate and/or of addition of the dimethylaminoethyl group onto dimethylaminoethyl methacrylate) in the same quantity, these compounds are nevertheless cleaved again in the subsequent stages, and so increase the selectivity for dimethylaminoethyl methacrylate to around 97%.

Below a catalyst concentration of 1 mol%, a drop in conversion of down to 95% is observed. In this concentration range, it becomes difficult to carry out uniform metering of the catalyst in solid form, this being so in particular for the poorly free-flowing LiOMe, and being the cause of the relatively wide scattering of the individual measurement values.

The aforesaid problems are eliminated by using a solution of 10% LiOMe in methanol. The additional methanol in the catalyst solution can be removed as an azeotrope from the first stage, without adversely affecting the overall reaction.

**Table 1:**

| **Catalyst LiNH₂** | | | | | | | | | | | | | | | |
|---|---|---|---|---|---|---|---|---|---|---|---|---|---|---|---|
| (mol%) Conversion Selectivity | 0.7% | 0.8% | 0.8% | 0.8% | 1.3% | 1.3% | 2.4% | 2.5% | 2.6% | 4.5% | 4.8% | 4.9% | 5.0% | 8.6% | 8.6% |
| | 94.9 | 95.9 | 95.6 | 95.7 | 96.3 | 96.5 | 96.9 | 96.9 | 96.7 | 97 | 97.4 | 96.7 | 97 | 98.2 | 98.3 |
| | 95.2 | 94.9 | 95.2 | 94.9 | 96.9 | 96.2 | 97.6 | 97.5 | 97.4 | 97.30 | 97.5 | 97.5 | 97.6 | 97.6 | 97.5 |

| **Catalyst LiOMe (solid)** | | | | | | | | | | | | | | | |
|---|---|---|---|---|---|---|---|---|---|---|---|---|---|---|---|
| (mol%) Conversion Selectivity | 0.4% | 0.8% | 0.8% | 0.8% | 1.3% | 1.4% | 1.5% | 1.5% | 2.0% | 2.1% | 4.6% | 4.6% | | | |
| | 91.2 | 96.3 | 96.9 | 97.5 | 98 | 97.9 | 95.9 | 97.4 | 97 | 97.2 | 96.1 | 96.4 | | | |
| | 95.6 | 96.1 | 96 | 96 | 95.7 | 96.1 | 97.1 | 97.5 | 97.2 | 96.7 | 96 | 96.6 | | | |

| **Catalyst LiOMe (solution)** | | | | | | | | | | | | | | | |
|---|---|---|---|---|---|---|---|---|---|---|---|---|---|---|---|
| (mol%) Conversion Selectivity | 0.8% | 0.8% | 1.3% | 1.4% | 2.3% | 2.3% | 4.7% | 4.7% | | | | | | | |
| | 96.1 | 96.3 | 98.1 | 97.8 | 97.1 | 97.4 | 95.9 | 96.1 | | | | | | | |
| | 95.7 | 95.2 | 95.6 | 96 | 97.1 | 96.6 | 95.9 | 95.4 | | | | | | | |

| **Catalyst LiOH / CaO** | 1:3.5 | | | 1:2.3 | | | | | | | | | | | |
|---|---|---|---|---|---|---|---|---|---|---|---|---|---|---|---|
| (mol%) Conversion Selectivity | 2.3% | 2.3% | | 3.5% | 3.4% | | | | | | | | | | |
| | 93.4 | 93.6 | | 93.6 | 94.5 | | | | | | | | | | |
| | 93.8 | 94.0 | | 94.0 | 94.0 | | | | | | | | | | |

| **Catalyst LiOH** | | | | | | | | | | | | | | | |
|---|---|---|---|---|---|---|---|---|---|---|---|---|---|---|---|
| (mol%) Conversion Selectivity | 2.1% | 3.9% | | | | | | | | | | | | | |
| | 96.2 | 97.4 | | | | | | | | | | | | | |
| | 94.2 | 94.0 | | | | | | | | | | | | | |

**Table 2:**

| | | | | | | |
|---|---|---|---|---|---|---|
| **Catalyst LiNH₂** | (mol%) | 0.8% | 1.3% | 2.5% | 4.9% | 8.6% |
| (averaged) | Conversion | 95.5 | 96.4 | 96.8 | 97.0 | 98.3 |
| (averaged) | Selectivity | 95.1 | 96.6 | 97.5 | 97.5 | 97.6 |
| | | | | | | |
| **Catalyst LiOMe** (solid) | (mol%) | 0.4% | 0.8% | 1.4% | 2.0% | 4.6% |
| (averaged) | Conversion | 91.2 | 96.9 | 97.3 | 97.1 | 96.3 |
| (averaged) | Selectivity | 95.6 | 96.0 | 96.6 | 97.0 | 96.3 |
| | | | | | | |
| **Catalyst LiOMe (solution)** | (mol%) | 0.8% | 1.3% | 2.3% | 4.7% | |
| (averaged) | Conversion | 96.2 | 98.0 | 97.3 | 96.0 | |
| (averaged) | Selectivity | 95.5 | 95.8 | 96.9 | 95.7 | |

## Claims

1. Process for preparing dimethylaminoalkyl (meth)acrylate, wherein a mixture comprising (a) alkyl (meth)acrylate, (b) dimethylaminoalkanol and (c) a catalyst system comprising a solution of a lithium alkoxide in alcohol is reacted, the method being **characterized in that** catalyst system contains no alkaline earth metal compounds.

2. Process according to Claim 1, **characterized in that** during the reaction further amounts of (a) alkyl (meth)acrylate, (b) dimethylaminoalkanol and optionally (c) catalyst system are added to the reaction mixture and dimethylaminoalkyl (meth)acrylate which forms is removed partly or completely from the reaction mixture.

3. Process according to Claim 1 or Claim 2, **characterized in that** the dimethylaminoalkanol is selected from the group consisting of 2-dimethylamino-1-ethanol, 3-dimethylamino-1-propanol, 4-dimethylamino-1-butanol, 5-dimethylamino-1-pentanol, 6-dimethylamino-1-hexanol, 7-dimethylamino-1-heptanol, and 8-dimethylamino-1-octanol.

4. Process according to any of Claims 1 to 3, **characterized in that** the alkyl (meth)acrylate is selected from the group consisting of methyl (meth)acrylate, ethyl(meth)acrylate, propyl (meth)acrylate, butyl (meth)acrylate, pentyl (meth)acrylate, hexyl (meth)acrylate, heptyl (meth)acrylate, and octyl (meth)acrylate.

5. Process according to any of Claims 1 to 4, **characterized in that** the dimethylaminoalkanol is 2-dimethylamino-1-ethanol and the alkyl (meth)acrylate is methyl methacrylate.

6. Process according to any of Claims 1 to 5, **characterized in that** the lithium alkoxide is selected from the group consisting of lithium methoxide, lithium ethoxide, lithium n-propoxide, lithium *iso*-propoxide, lithium n-butoxide, lithium *iso*-butoxide and lithium *tert-*butoxide, and **in that** the alcohol independently thereof is selected from the group consisting of methanol, ethanol, n-propanol, *iso*-propanol, n-butanol, *iso*-butanol and *tert-*butanol.

7. Process according to any of Claims 1 to 6, **characterized in that** the catalyst system consists of a solution of lithium methoxide in methanol or of a solution of lithium *tert-*butoxide in methanol or *tert*-butanol.

8. Process according to any of Claims 1 to 7, **characterized in that** the reaction mixture is heated to a temperature in the range from 100 to 140°C.

9. Process according to any of Claims 1 to 8, **characterized in that** the reaction mixture further comprises one or more inhibitors selected from the group consisting of hydroquinone monomethyl ether and 2,4-dimethyl-6-tert-butylphenol.

10. Process according to any of Claims 1 to 9, **characterized in that** the molar ratio of (a) alkyl (meth)acrylate to (b) dimethylaminoalkanol in the reaction mixture is 3.5:1 to 1.1:1.

11. Process according to any of Claims 1 to 10, **characterized in that** the fraction of lithium alkoxide in the reaction mixture is 0.4 to 5 mol%, based on the dimethylaminoalkanol.

## Patentansprüche

1. Verfahren zur Herstellung von Dimethylaminoalkyl-(meth)acrylat, bei dem eine Mischung, umfassend (a) Alkyl(meth)acrylat, (b) Dimethylaminoalkanol und (c) ein Katalysatorsystem, das eine Lösung eines Lithiumalkoxids in Alkohol umfasst, zur Reaktion gebracht wird, wobei das Verfahren **dadurch gekennzeichnet ist, dass** das Katalysatorsystem keine Erdalkalimetallverbindungen enthält.

2. Verfahren nach Anspruch 1, **dadurch gekennzeichnet, dass** während der Reaktion weitere Mengen von (a) Alkyl(meth)acrylat, (b) Dimethylaminoalkanol und gegebenenfalls (c) Katalysatorsystem zur Reaktionsmischung zugegeben werden und sich bildendes Dimethylaminoalkyl(meth)acrylat teilweise oder vollständig aus der Reaktionsmischung entfernt wird.

3. Verfahren nach Anspruch 1 oder Anspruch 2, **dadurch gekennzeichnet, dass** das Dimethylaminoalkanol aus der Gruppe bestehend aus 2-Dimethylamino-1-ethanol, 3-Dimethylamino-1-propanol, 4-Dimethyl-amino-1-butanol, 5-Dimethylamino-1-pentanol, 6-Dimethylamino-l-hexanol, 7-Dimethylamino-1-heptanol und 8-Dimethylamino-1-octanol ausgewählt wird.

4. Verfahren nach einem der Ansprüche 1 bis 3, **dadurch gekennzeichnet, dass** das Alkyl(meth)-acrylat aus der Gruppe bestehend aus Methyl(meth)-acrylat, Ethyl(meth)acrylat, Propyl(meth)acrylat, Butyl(meth)acrylat, Pentyl(meth)acrylat, Hexyl-(meth)acrylat, Heptyl(meth)acrylat und Octyl-(meth)acrylat ausgewählt wird.

5. Verfahren nach einem der Ansprüche 1 bis 4, **dadurch gekennzeichnet, dass** das Dimethylaminoalkanol 2-Dimethylamino-1-ethanol ist und das Alkyl(meth)acrylat Methylmethacrylat ist.

6. Verfahren nach einem der Ansprüche 1 bis 5, **dadurch gekennzeichnet, dass** das Lithiumalkanolat aus der Gruppe bestehend aus Lithiummethanolat, Lithiumethanolat, Lithium-n-propanolat, Lithiumisopropanolat, Lithium-n-butanolat, Lithiumisobutanolat und Lithium-tert-butanolat ausgewählt wird und dass der Alkohol unabhängig davon aus der Gruppe bestehend aus Methanol, Ethanol, n-Propanol, Isopropanol, n-Butanol, Isobutanol und tert-Butanol ausgewählt wird.

7. Verfahren nach einem der Ansprüche 1 bis 6, **dadurch gekennzeichnet, dass** das Katalysatorsystem aus einer Lösung von Lithiummethanolat in Methanol oder einer Lösung von Lithium-tert-butanolat in Methanol oder tert-Butanol besteht.

8. Verfahren nach einem der Ansprüche 1 bis 7, **dadurch gekennzeichnet, dass** die Reaktionsmischung auf eine Temperatur im Bereich von 100 bis 140 °C aufgeheizt wird.

9. Verfahren nach einem der Ansprüche 1 bis 8, **dadurch gekennzeichnet, dass** die Reaktionsmischung weiterhin einen oder mehrere Inhibitoren ausgewählt aus der Gruppe bestehend aus Hydrochinonmonomethylether und 2,4-Dimethyl-6-tert-butylphenol enthält.

10. Verfahren nach einem der Ansprüche 1 bis 9, **dadurch gekennzeichnet, dass** das Molverhältnis von (a) Alkyl(meth)acrylat zu (b) Dimethylaminoalkanol in der Reaktionsmischung 3,5:1 bis 1,1:1 beträgt.

11. Verfahren nach einem der Ansprüche 1 bis 10, **dadurch gekennzeichnet, dass** der Anteil an Lithiumalkanolat in der Reaktionsmischung 0,4 bis 5 Mol-%, bezogen auf das Dimethylaminoalkanol, beträgt.

## Revendications

1. Procédé de préparation de (méth)acrylate de diméthylaminoalkyle, dans lequel un mélange comprenant (a) un (méth)acrylate d'alkyle, (b) un diméthylaminoalcanol et (c) un système de catalyseur comprenant une solution d'un alcoxyde de lithium dans un alcool réagit, le procédé étant **caractérisé en ce que** le système de catalyseur ne contient aucun composé de métal alcalino-terreux.

2. Procédé selon la revendication 1, **caractérisé en ce que** pendant la réaction, des quantités supplémentaires de (a) (méth)acrylate d'alkyle, (b) diméthylaminoalcanol et facultativement (c) système de catalyseur sont ajoutées au mélange de réaction et le (méth)acrylate de diméthylaminoalkyle qui se forme est partiellement ou complètement retiré du mélange de réaction.

3. Procédé selon la revendication 1 ou la revendication 2, **caractérisé en ce que** le diméthylaminoalcanol est choisi dans le groupe constitué des 2-diméthylamino-1-éthanol, 3-diméthylamino-1-propanol, 4-diméthylamino-1-butanol, 5-diméthylamino-1-pentanol, 6-diméthylamino-1-hexanol, 7-diméthylamino-1-heptanol et 8-diméthylamino-1-octanol.

4. Procédé selon l'une quelconque des revendications 1 à 3, **caractérisé en ce que** le (méth)acrylate d'alkyle est choisi dans le groupe constitué des (méth)acrylate de méthyle, (méth)acrylate d'éthyle, (méth)acrylate de propyle, (méth)acrylate de butyle, (méth)acrylate de pentyle, (méth)acrylate d'hexyle, (méth)acrylate d'heptyle et (méth)acrylate d'octyle.

5. Procédé selon l'une quelconque des revendications 1 à 4, **caractérisé en ce que** le diméthylaminoalcanol est le 2-diméthylamino-1-éthanol et le (méth)acrylate d'alkyle est le méthacrylate de méthyle.

6. Procédé selon l'une quelconque des revendications 1 à 5, **caractérisé en ce que** l'alcoxyde de lithium est choisi dans le groupe constitué des méthoxyde de lithium, éthoxyde de lithium, n-propoxyde de lithium, *iso-*propoxyde de lithium, n-butoxyde de lithium, iso-butoxyde de lithium et tert-butoxyde de lithium, et **en ce que** l'alcool, indépendamment de celui-ci, est choisi dans le groupe constitué des méthanol, éthanol, n-propanol, *iso-*propanol, n-butanol, iso-butanol et tert-butanol.

7. Procédé selon l'une quelconque des revendications 1 à 6, **caractérisé en ce que** le système de catalyseur est constitué d'une solution de méthoxyde de lithium dans le méthanol ou d'une solution de tert-butoxyde de lithium dans le méthanol ou le tert-butanol.

8. Procédé selon l'une quelconque des revendications 1 à 7, **caractérisé en ce que** le mélange de réaction est chauffé à une température dans la plage de 100 à 140 °C.

9. Procédé selon l'une quelconque des revendications 1 à 8, **caractérisé en ce que** le mélange de réaction comprend en outre un ou plusieurs inhibiteurs choisis dans le groupe constitué de l'éther monométhylique d'hydroquinone et du 2,4-diméthyl-6-tert-butylphénol.

10. Procédé selon l'une quelconque des revendications 1 à 9, **caractérisé en ce que** le rapport molaire du (a) (méth)acrylate d'alkyle au (b) diméthylaminoalcanol dans le mélange de réaction est de 3,5:1 à 1,1:1.

11. Procédé selon l'une quelconque des revendications 1 à 10, **caractérisé en ce que** la fraction d'alcoxyde de lithium dans le mélange de réaction est de 0,4 à 5 % en moles, sur la base du diméthylaminoalcanol.
